# EUROPEAN PATENT SPECIFICATION

(11) **EP 3 127 514 B1**
(45) Date of publication and mention of the grant of the patent: **25.11.2020**
(21) Application number: 15773253.8
(22) Date of filing: 03.04.2015
(51) Int. Cl.: A61B 17/88, A61B 34/30, A61B 34/35, A61B 17/64, A61F 5/04

(54) **BONE RETRACTION DEVICE AND FRACTURE REDUCTION SYSTEM INCLUDING SAME**
KNOCHENRETRAKTIONSVORRICHTUNG UND REDUKTIONSSYSTEM DAMIT
DISPOSITIF DE RÉTRACTION OSSEUSE ET SYSTÈME DE RÉDUCTION DE FRACTURE LE COMPRENANT

(30) Priority: 03.04.2014 KR 20140040001
(43) Date of publication of application: 08.02.2017
(73) Proprietor: Kyungpook National University Industry-Academic Cooperation Foundation, Daegu 41566 (KR)
(72) Inventor: PARK, Il-hyung, Daegu 706-776 (KR); JOUNG, Sang-hyun, Daegu 702-843 (KR); PARK, Chul-woo, Daegu 706-788 (KR); OH, Chang-wuk, Daegu 706-794 (KR)
(74) Representative: MacDougall, Alan John Shaw
(86) International application number: PCT/KR2015/003373
(87) International publication number: WO 2015/152679

(56) References cited:
- CN-A- 102 283 731
- GB-A- 1 191 345
- KR-A- 20000 011 134
- KR-A- 20010 065 553
- KR-A- 20010 065 553
- KR-B1- 100 391 252
- KR-Y1- 200 335 336
- US-A- 5 380 322
- US-A1- 2010 312 291
- US-A1- 2011 172 664

## Description

### [Technical Field]

The present disclosure relates to a bone traction device and a fracture reduction system including the same, and more particularly, to a bone traction device used for bonding the respective fractured bone fragments of a patient and a fracture reduction system including the same.

### [Background Art]

A surgical procedure on arm, leg, and pelvis fractures largely consists of a reduction and a fixing of bone fragments. Two to three surgeons generally participate in the surgical procedure. The reason why many surgeons are required is that there is a need to tow arm, leg, and pelvis portions (hereinafter, referred to as fractured portions) with a great force of about 200 to 400 N for the fracture reduction.

By the way, the fractured portion of the patient is towed and then the towed position needs to be kept, which puts a great physical burden on surgeons. Meanwhile, allocating many surgeons to perform only the operations for which a physical labor is required may be reckoned as a form of inefficient manpower management.

Meanwhile, a fracture table used only for fracture surgery for the foregoing traction has been marketed. However, since the fracture table is a form in which the fractured portion is towed while fixing a foot portion, it is difficult to accurately adjust positions of bone fragments and measure a force required for traction of bone fragments, which may cause an excessive traction.

Further, tries to solve the above problems using a surgical navigation and a robot have been conducted. In this case, a traction force applied to the robot is very large and therefore a volume of the robot needs to be increased to satisfy the traction force. As a result, the robot may not meet a compact design required for an operating room.

KR 2001 0065553 discloses an apparatus for longitudinally extending fractured bones and restoring the bones to the original position. The apparatus comprises a first support part having a first support device installed thereto to be moved in diverse directions by a vertical moving device, a front-and-rear moving device and a horizontal moving device for rotating and fixing one side of fractured bone, and a second support part having a second support device installed thereto to be moved on the earth axis for fixing the other side of the fracture bone.

US 2011/0172664 discloses an orthopaedic device for correcting deformities of a long bone, comprising a bar, extended along an axis and intended to be placed alongside the bone, at least a first clamp for a first group of osseous screws and a second clamp for a second group of osseous screws, removably mounted on said bar, wherein the first of said clamps is placed onboard a support base in turn mounted on said bar, and angularly movable in relation to the support base around a given rotation axis by means of a rotary coupling. The coupling comprises a male element associated with the first clamp and having a surface at least partially cylindrical, and a corresponding female element associated with the support base and having a surface at least partially cylindrical constituting a seat for the loose coupling of the male element.

US 5,380,322 discloses a bone fixation device comprising a profiled bar on which at least two supporting members have been movably and lockably mounted. The supporting members comprise a sliding portion having an opening therein for passing the bar and a supporting plate extending from the sliding part, with a convex and concave surface. Against the convex surface lies a ring and against the concave surface lies a convex surface of a receiving element in which screws have been clamped, which can be secured in a bone part. Through the ring extends a bolt that has been screwed into one of a number of threaded bores in the receiving element and which bolt extends through a relatively large opening in the supporting plate.

### [Disclosure]

### [Technical Problem]

The present disclosure provides a bone traction device that can facilitate an accurate reduction of bone fragments and be compactly manufactured because a fine adjustment of the bone fragments towed is freely performed even in a state in which a traction force is applied to fractured portions (arm, leg, and pelvis portions) of a patient at the time of directly or indirectly towing the fractured portions of the patient, and a fracture reduction system including the same.

### [Technical Solution]

The present invention provides a bone traction device and a fracture reduction apparatus, as set out in the appended claims.

The bone traction device includes:
a base; a support vertically disposed on the base; a traction shaft provided at the support in a perpendicular direction and adapted to be driven in forward and backward directions; and a double-joint part connected to the traction shaft and adapted to be bent in multiple stages with respect to a driving direction of the traction shaft.

The double-joint part includes: a first ball joint pivotally connected to one end portion of the traction shaft; a second ball joint spaced apart from the first ball joint; a connection member interconnecting the first and second ball joints; and a socket member pivotally connected with the second ball joint and connected to a patient.

The connection member may be formed of a metal bar or a wire having stiffness.

The bone traction device may further include: a gripping member installed in the socket member and griping bone fragments of the patient. The gripping member may be a C type clamp.

The bone traction device may further include: air boots enclosing a part of a fractured portion of the patient in a pressed state, in which the socket member may be connected to one end portion of the air boots.

The traction shaft may include a dynamics sensor for detecting a traction force. The bone traction device may further include: a display electrically connected to the dynamics sensor to display the refraction force detected by the dynamics sensor.

The traction shaft may include a fail-safe part.

The support may be driven to be vertically elevated to set a height of the traction shaft.

The fracture reduction apparatus includes: a first bone fragment support part for gripping a proximal bone fragment; a second bone fragment support part for gripping a distal bone fragment; and a bone traction device as claimed in claim 1 for towing a fractured portion of a patient, wherein a tip portion of the bone traction device is formed to be bendable to change a position of the distal bone fragment gripped by the second bone fragment support part.

The first and second bone fragment support parts may each be provided with a pair of fixing pins; and at least one spike that guides the pair of fixing pins to be fixed to the respective bone fragments and is fixed to the respective bone fragments at a tip portion of the first and second bone fragment.

A stopper to restrict an insertion depth of the respective fixing pins into the respective bone fragments may protrude at outer circumferences of the tip portions of the pair of fixing pins, respectively.

The refraction reduction system may further include: a remote controller remotely controlling a driving of the bone traction device.

### [Advantageous Effects]

As described above, according to the exemplary embodiments of the present disclosure, it is possible to perform the accurate bone fragment osteosynthesis because the adoption of the double-joint part freely performs the fine adjustment of the bone fragments towed even in the state in which the traction force is applied to the fractured portions (arm, leg, and pelvis portions) of the patient at the time of directly or indirectly towing the fractured portions of the patient.

Further, according to the exemplary embodiments of the present disclosure, since the bone traction device serves only to tow the fractured portions (arm, leg, and pelvis portions) of the patient, the size of the bone traction device may be compactly kept, and as a result the bone traction device may be easily installed in the operating room.

### [Description of Drawings]

FIG. 1 is a diagram schematically illustrating a fracture reduction system according to a first exemplary embodiment of the present disclosure;
FIG. 2 is a perspective view illustrating a bone traction device illustrated in FIG. 1;
FIG. 3 is a side view illustrating a state in which a lower bone fragment is towed by the bone traction device illustrated in FIG. 1;
FIG. 4 is a perspective view illustrating a state in which a pair of fixing pins are fixed to a proximal bone fragment and a distal bone fragment, respectively, illustrated in FIG. 1;
FIG. 5 is a diagram schematically illustrating a fixed angle of the pair of fixing pins illustrated in FIG. 4 that are fixed to the bone fragment;
FIGS. 6 and 7 are diagrams schematically illustrating the fixing pin guided to a sleeve to be fixed to the bone fragment;
FIG. 8 is a diagram schematically illustrating a simple fixing apparatus installed on the bone fragment after a reduction operation is performed;
FIG. 9 is a diagram schematically illustrating a fracture reduction system according to a second exemplary embodiment of the present disclosure;
FIG. 10 is a diagram schematically illustrating a fracture reduction system according to a third exemplary embodiment of the present disclosure; and
FIG. 11 is a diagram schematically illustrating a fracture reduction system according to a fourth exemplary embodiment of the present disclosure.

### [Mode for Invention]

Hereinafter, exemplary embodiments of the present disclosure will be described with reference to the accompanying drawings. Embodiments described below are exemplarily described to help understanding of the present disclosure and therefore it is to be understood that the present disclosure may be various changed differently from the embodiments described herein. However, in describing the present disclosure, if it is determined that the detail description of relevant known functions or components makes subject matters of the present disclosure obscure, the detailed description and illustration thereof will be omitted. Further, to help understanding of the present disclosure, the accompanying drawings are not necessarily illustrated to scale but dimensions of some components may be illustrated to be exaggerated.

Hereinafter, a fracture reduction system according to first to fourth exemplary embodiments of the present disclosure may be applied to all fractured portions besides arm and leg portions but in the present embodiment, the leg fracture will be described as an example.

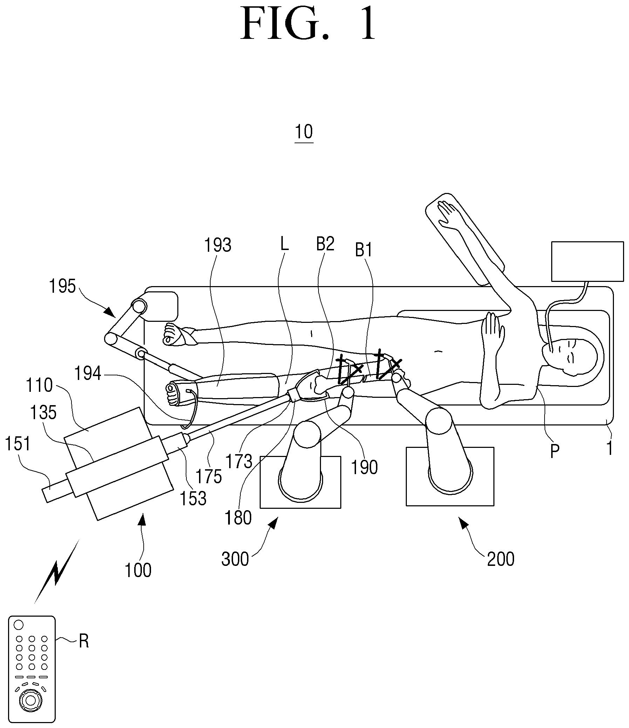

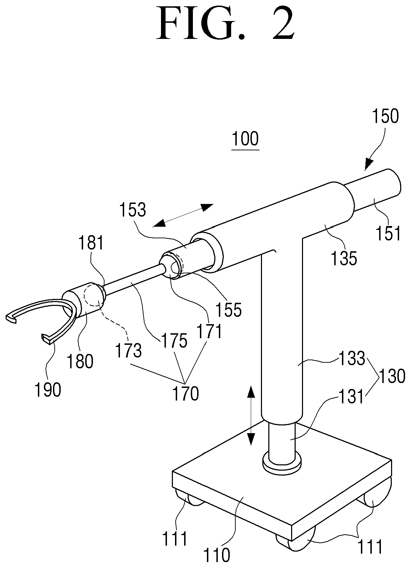

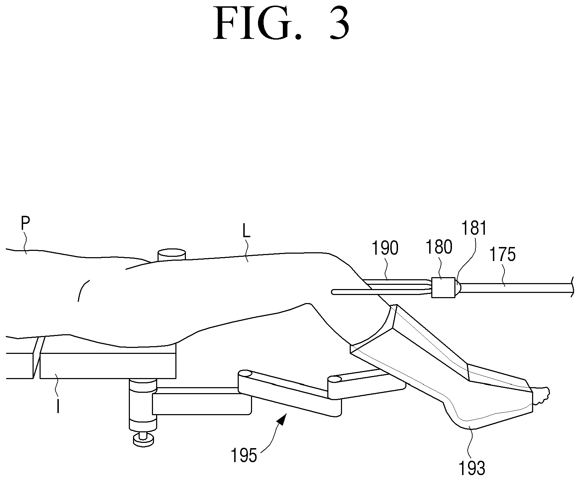

FIG. 1 is a diagram schematically illustrating a fracture reduction system according to a first exemplary embodiment of the present disclosure, FIG. 2 is a perspective view illustrating a bone traction device illustrated in FIG. 1, and FIG. 3 is a side view illustrating a state in which a lower bone fragment is towed by the bone traction device illustrated in FIG. 1.

Referring to FIG. 1, a fracture reduction system 10 according to a first exemplary embodiment of the present disclosure includes a bone traction device 100 towing a fractured portion of a patient P, air boots 193 enclosing a fractured portion, a first bone fragment support part 200 supporting a proximal bone fragment B1, and a second bone fragment support part 300 supporting a distal bone fragment B2.

The bone traction device 100 directly tows the distal bone fragment B2 but has a structure in which a part of the bone traction device 100 is pivoted in a multi stage to give a degree of freedom to a second bone fragment support part 300 supporting the distal bone fragment B2 to be able to move the distal bone fragment B2 to a predetermined position. Further, the bone traction device 100 may provide a sufficient traction force (for example, 200 to 400 N) towing the distal bone fragment B2 at the time of a reduction operation while being compactly formed.

The bone traction device 100 as described above includes a base 110, a support 130, a traction shaft 150, a double-joint part 170, a socket member 180, and a gripping member 190.

Referring to FIG. 2, the base 110 is seated around a surgery bed 1 and a bottom thereof is provided with a plurality of wheels 111 for moving the base 110. The plurality of wheels 111 each preferably include a general locking structure (not illustrated) for selectively locking a rotation of the wheels 111.

The support 130 is installed to be perpendicular to the base 110. In this case, the support 130 is configured as a general hydraulic actuator or pneumatic actuator or an electric or manual actuator to be driven up and down. For example, the support 130 may include a fixed part 131 of which the lower end portion is fixed to an upper surface of the base 110 and a movable part 133 coupled to the fixed part 131.

Further, an upper end portion of the support 130 is integrally formed with a shaft support part 135 for supporting a traction shaft 150. The shaft support part 135 is disposed in approximately a perpendicular direction (horizontal direction with respect to a ground) with respect to the movable part 133.

The traction shaft 150 is slidably installed in the shaft support part 135 to be driven in forward and backward directions. The traction shaft 150 may be driven by being supplied with a driving force by the general hydraulic actuator or pneumatic or electric or manual actuator connected to a back end portion 151 of the traction shaft 150.

The traction shaft 150 may tow the distal bone fragment B2 with a predetermined traction force. In this case, the traction shaft 150 may be provided with a dynamics sensor (not illustrated) for detecting a traction force, in which the dynamics sensor may be, for example, a mechanical pressure sensor.

Further, the fracture reduction system 10 according to the first exemplary embodiment of the present disclosure may be electrically connected to a display (not illustrated) that displays the traction force detected by the dynamics sensor in a numerical value and enables a medical team to check the refraction force.

Further, the traction shaft 150 may include a fail-safe part (not illustrated). Stabilizers such as the fail-safe part are to prevent soft tissues such as a nerve, a blood vessel, a muscle, and a ligament from being damaged due to the traction force provided by the traction shaft 150.

The double-joint part 170 is pivotally connected to a tip portion of the traction shaft 150 in multiple stages with respect to a driving direction of the traction shaft 150. Even though the distal bone fragment B2 is being towed by the double-joint part 170, the second bone fragment support part 300 is given a degree of freedom to be able to move the distal bone fragment B2 to some extent.

Therefore, the distal bone fragment B2 may move to an accurate osteosynthesis position with the proximal bone fragment B1 by a fine adjustment of the second bone fragment support part 300. At this point, the great traction force for driving the second bone fragment support part 300 is not required, and therefore as the second bone fragment support part 300, the existing 6-axis robot may also be adopted.

As described above, the double-joint part 170 includes a first ball joint 171, a second ball join 173, and a connection member 175.

The first ball joint 171 is pivotally connected to a joint groove 155 formed at a tip portion 153 of the traction shaft 150. The joint groove 155 may have approximately a spherical shape corresponding to a shape of the first ball joint 171.

The second ball joint 173 is pivotally connected to a joint groove 181 formed at a back end portion of the socket member 180. The joint groove 181 of the socket member 180 may also have approximately a spherical shape corresponding to the shape of the second ball joint 171.

The connection member 175 interconnects between the first and second ball joints 171 and 173 and may be formed of a metal bar or a metal wire having predetermined stiffness.

The joint groove 181 formed at one end portion of the socket member 180 is pivotally connected to the second ball joint 173 as described above. Further, the other end portion of the socket member 180 is fixedly provided with the gripping member 190.

The gripping member 190 may be formed of approximately a C-type clamp that fixes both sides of the end portion of the distal bone fragment B2. Meanwhile, to reduce the fractured portion, in some cases, a strong traction force is required, but when the ligament of the patient P is damaged, the traction through a joint may be impossible. In this case, the distal bone fragment B2 may be towed through the traction shaft 153 by directly connecting the gripping member 190 to the distal bone fragment B2

The air boots 193 enclose approximately a knee of the fractured portion of the patient P and presses a leg with an air pressure supplied through an air supply hose 194 and fixes the leg. The air boots 193 serve to prevent a thrombus and serves as a foot rest.

The air boots 193 are supported by an air boots fixing arm 195 that is formed of three links to support a self weight of a leg and has 3 degrees of freedom. In this case, the air boots fixing arm 195 is fixed to a portion of the surgery bed 1.

Referring back to FIG. 1, a medical team may remotely operate the bone traction device 100 using a remote controller R. The medical team may tow the distal bone fragment B2 while checking a 2D or 3D perspective image without being exposed by radiation by using a radiation shielding film (not illustrated) at the time of operating the remote controller R.

As such, the number of persons required for the surgery may be minimized by operating the remote controller R and if there is abnormality in a radio operation of the bone traction device 100 using the remote controller R, a direct command to manually operate the bone traction device 100 may be issued. Therefore, the present disclosure may improve reliability of the fracture reduction system.

The first bone fragment support part 200 supports the proximal bone fragment B1 and the second bone fragment support part 300 supports the distal bone fragment B2. The first and second bone fragment support parts 200 and 300 both may be formed of a multi-axis robot having multi degrees of freedom. For example, the first and second bone fragment support parts 200 and 300 may be formed of a 6 axis robot having 6 degrees of freedom.

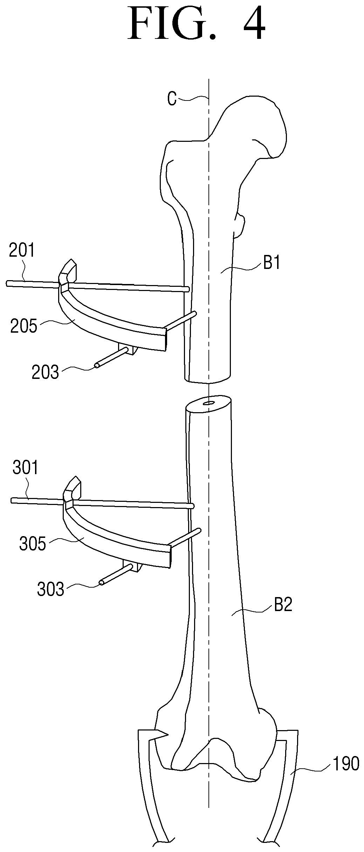

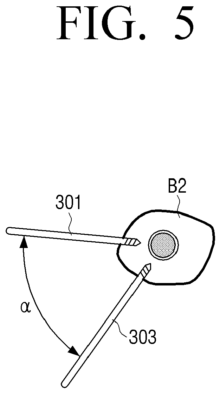

Referring to FIG. 4, each end portion of the multi-axis robot is provided with a pair of fixing pins 201 and 203 and 301 and 303 and arched support frames 205 and 305 supporting them. In this case, as illustrated in FIG. 5, the pair of fixing pins 301 and 303 is fixed to the respective bone fragments at an angle of about 60 ° or more to increase the fixing force and is preferably set to be spaced apart from each other by approximately 3 cm or more in parallel with the bone shaft C.

Meanwhile, after the fracture is reduced, a predetermined nail (not illustrated) is inserted between the bone fragments B1 and B2 or a metal plate (not illustrated) is used to fix between the bone fragments B1 and B2. To this end, the existing method of completely penetrating a fixing pin through a bone fragment has any problem, and therefore it is preferable to penetrate a fixing pin through only a cortex of one side of a bone as illustrated in FIG. 5.

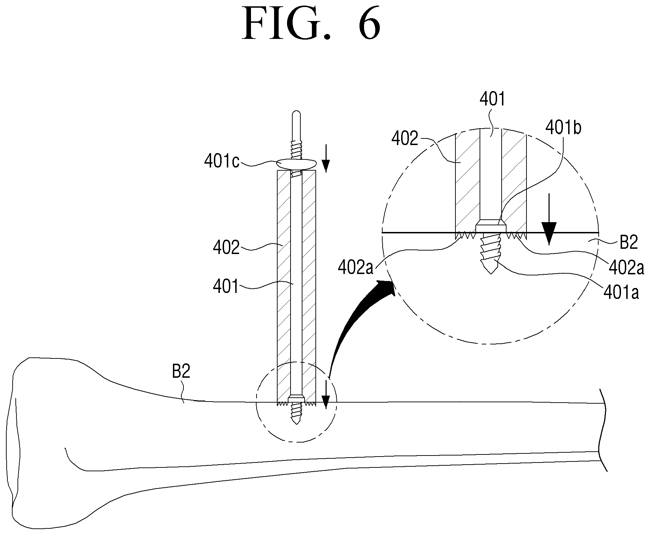

Referring to FIG. 6, as described above, a stopper 401b protrudes at a circumference of a tip portion 401a of a fixing pin 401, and thus an insertion depth of the fixing pin 401 into the bone fragment B2 may be easily controlled.

In this case, a sleeve 402 is fixed to the bone fragment before the tip portion 401a of the fixing pin 401 is inserted into the bone fragment B2. In this state, the fixing pin 401 is inserted into the sleeve 402 to be guided to the sleeve 402.

A tip portion of the sleeve 402 is provided with a plurality of circular spikes 402a that are fixed to the cortex of the bone fragment B2. Therefore, the sleeve 402 may firmly support the fixing pin 401. In this case, a hanging protrusion 401c having approximately a flange shape protrudes at a back end portion of the fixing pin 401 to prevent the sleeve 402 from being separated from the fixing pin 401.

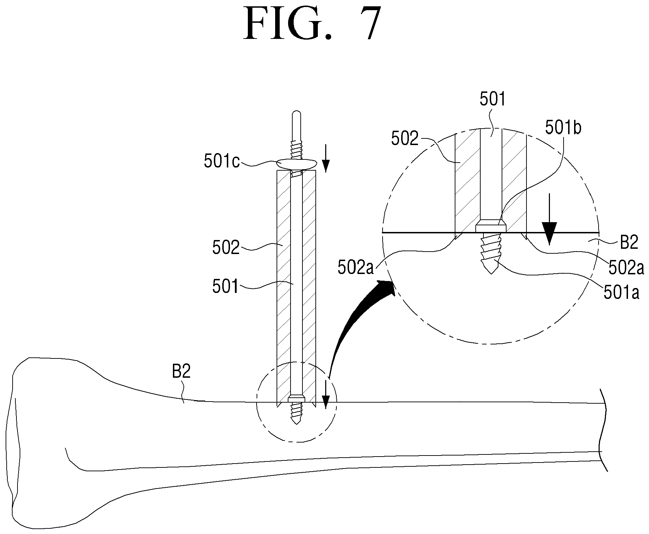

Referring to FIG. 7, a fixing pin 501 and a sleeve 502 are formed to be identical with the fixing pin 401 and the sleeve 402 illustrated in FIG. 6. However, there is a difference in a fact that a circular spike 502a formed at a tip portion of the sleeve 502 is integrally formed.

In FIG. 7, non-explained reference numerals 501b and 501c each indicate a stopper and a hanging protrusion.

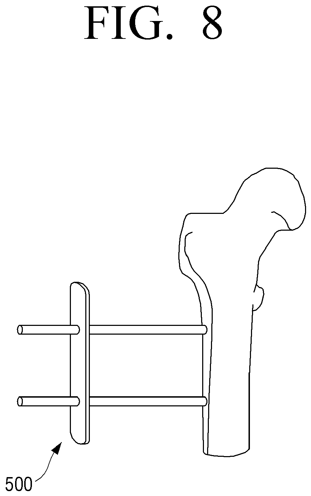

Meanwhile, after the fracture reduction is completed by the fracture reduction system 10 according to the embodiment of the present disclosure, if necessary, as illustrated in FIG. 8, a simple fixing apparatus 500 may be used to fix between the bone fragments B1 and B2 and may be used to disconnect the connection between the first and second bone fragment support parts 200 and 300 and then fix the bone fragments B1 and B2.

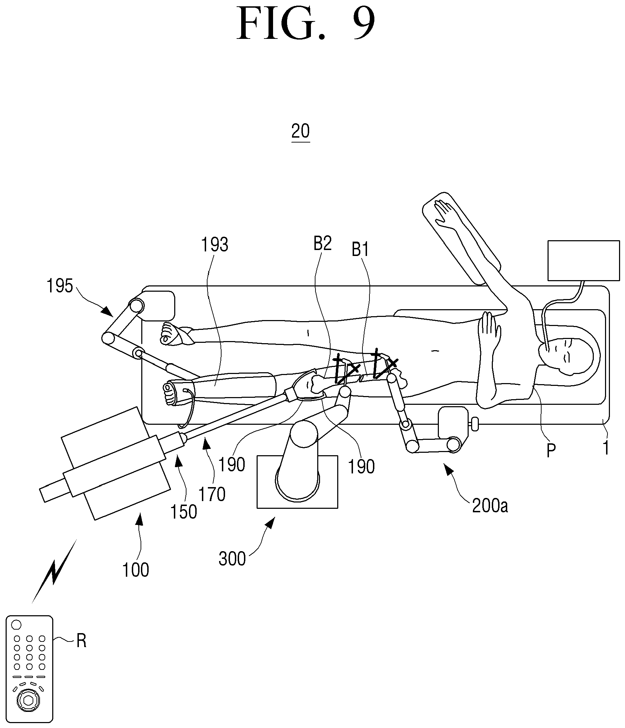

FIG. 9 is a diagram schematically illustrating a fracture reduction system according to a second exemplary embodiment of the present disclosure. A fracture reduction system 20 illustrated in FIG. 9 is configured to have substantially the same configuration as the fracture reduction system 10 according to the first exemplary embodiment of the present disclosure. However, there is a difference in the fact that the first bone fragment support part 200a is formed of a multi-axis passive arm having a smaller size than the multi-axis robot instead of the multi-axis robot.

The reason of applying the multi-axis passive-arm is to consider the fact that the movement of the proximal bone fragment B1 is smaller than that of the distal bone fragment B2 during the reduction surgery, which has an advantage of making the fracture reduction system cheap and ensuring a wide surgery space.

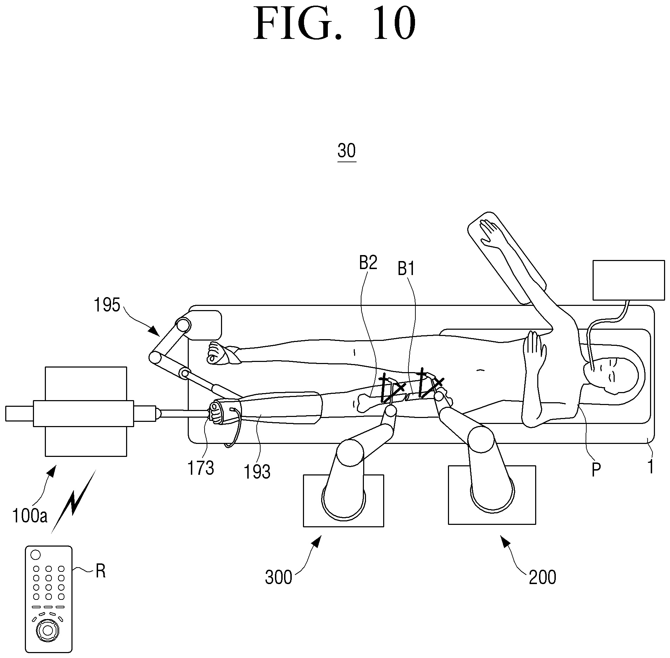

FIG. 10 is a diagram schematically illustrating a fracture reduction system according to a third exemplary embodiment of the present disclosure. A fracture reduction system 30 illustrated in FIG. 10 has substantially the same configuration as the fracture reduction system 10 according to the first exemplary embodiment of the present disclosure as described above. However, there is a difference in the fact that a bone traction device 100a does not directly grip the distal bone fragment B2 but the socket member 173 is fixed to a portion of the air boots 193.

As such, the indirect traction of the distal bone fragment B2 through the air boots 193 may be applied to the case in which the ligament of the patient P is not damaged or a bone density of the fractured bone fragment is reduced due to osteoporosis. As such, when the bone traction device 100a is directly connected to the air boots 193 without the gripping member 190, it is possible to ensure a wider surgery space.

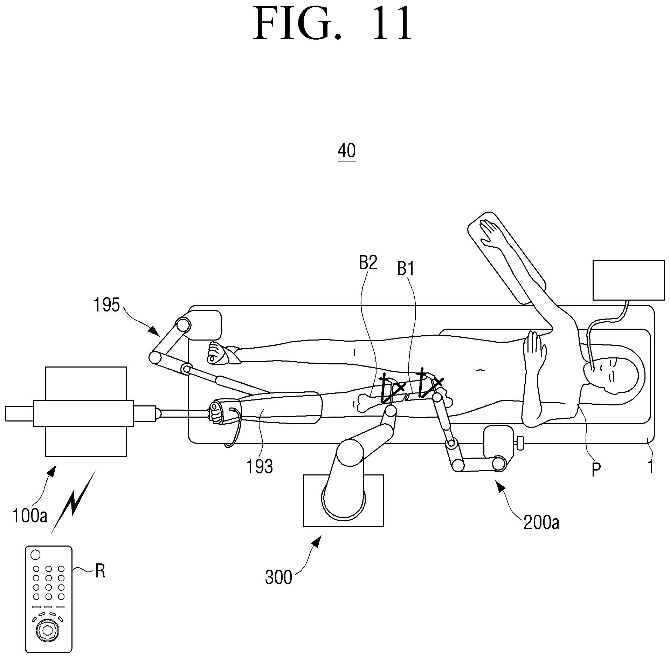

FIG. 11 is a diagram schematically illustrating a fracture reduction system according to a fourth exemplary embodiment of the present disclosure. A fracture reduction system 40 illustrated in FIG. 11 is configured to have substantially the same configuration as the fracture reduction system 30 according to the third exemplary embodiment of the present disclosure. However, there is a difference in the fact that the first bone fragment support part 200a is formed of a multi-axis passive-arm having a smaller size than the multi-axis robot instead of the multi-axis robot.

As such, the fracture reduction systems 10, 20, 30, and 40 according to the present disclosure may appropriately replace the first bone fragment support parts 200 and 200a by the multi-axis robot or the multi-axis passive-arm depending on the state of the fractured portion (arm, leg, or pelvis portion) or directly tow the distal bone fragment B2 by the bone traction devices 100 and 100a or may be changed to indirectly tow the distal bone fragment B2 by towing the air boots 193.

As described above, although the present disclosure has been described with reference to exemplary embodiments and the accompanying drawings, the present disclosure is not limited thereto, but may be variously modified and altered by those skilled in the art to which the present disclosure pertains without departing from the scope of the present disclosure The scope of the present invention is defined by the claims.

### [Industrial Applicability]

The present disclosure relates to a bone traction device for bonding the respective fractured bone fragments of the patient and the fracture reduction system including the same.

## Claims

1. A bone traction device (100), comprising:
a base (110);
a support (130) vertically disposed on the base (110);
a traction shaft (150) provided at the support (130) in a perpendicular direction and
adapted to be driven in forward and backward directions; **characterised by**
a double-joint part (170) connected to the traction shaft (150) and adapted to be bent in multiple stages with respect to a driving direction of the traction shaft (150),
wherein the double-joint part (170) includes: a first ball joint (171) pivotally connected to one end portion of the traction shaft (150); a second ball joint (173) spaced apart from the first ball joint (171); a connection member (175) interconnecting the first and second ball joints; and a socket member (180) pivotally connected with the second ball joint (173) and connected to a patient.

2. The bone traction device (100) as claimed in claim 1, wherein the connection member (175) is formed of a metal bar having stiffness or a wire having stiffness.

3. The bone traction device (100) as claimed in claim 1, further comprising: a gripping member (190) installed in the socket member (180) to grip bone fragments of the patient.

4. The bone traction device (100) as claimed in claim 3, wherein the gripping member (190) is a C type clamp.

5. The bone traction device (100) as claimed in claim 1, further comprising: air boots (193) enclosing a part of a fractured portion of the patient with pressure, wherein the socket member (180) is connected to one end portion of the air boots (193).

6. The bone traction device (100) as claimed in claim 1, wherein the traction shaft (150) includes a fail-safe part.

7. The bone traction device (100) as claimed in claim 1, wherein the support (130) is adapted to be driven to be vertically elevated or descended to set a height of the traction shaft (150).

8. A fracture reduction apparatus, comprising:
a first bone fragment support part (200) for gripping a proximal bone fragment (B1);
a second bone fragment support part (300) for gripping a distal bone fragment (B2); and
a bone traction device (100) as claimed in claim 1, for towing a fractured portion of a patient, wherein a tip portion of the bone traction device (100) is formed to be bendable to change a position of the distal bone fragment (B2) gripped by the second bone fragment support part (300).

9. The fracture reduction apparatus as claimed in claim 8, further comprising a gripping member (190) installed in the socket member (180) to grip bone fragments of the patient.

10. The fracture reduction apparatus as claimed in claim 8, further comprising:
air boots (193) enclosing a part of a fractured portion of the patient in a pressed state, wherein the socket member (180) is connected to one end portion of the air boots (193).

11. The fracture reduction apparatus as claimed in claim 8, wherein the first bone fragment support part (200) is a multi-axis robot having multi degrees of freedom at the proximal bone fragment (B1) and the second bone fragment support part (300) is a multi-axis robot having multi degrees of freedom at the distal bone fragment (B2).

12. The fracture reduction apparatus as claimed in claim 8, wherein the first bone fragment support part (200) is a multi-axis passive arm fixed to a surgery bed and having multi degrees of freedom at the proximal bone fragment (B1) and the second bone fragment support part (200) is a multi-axis robot having multi degrees of freedom at the distal bone fragment (B2).

13. The fracture reduction apparatus as claimed in claim 8, wherein each of the first and second bone fragment support parts is provided with a pair of fixing pins (201, 203); and at least one spike that guides the pair of fixing pins (201, 203) to be fixed to the respective bone fragments and is fixed to the respective bone fragments at a tip portion of the first and second bone fragment support parts.

## Patentansprüche

1. Knochentraktionsvorrichtung (100), die Folgendes umfasst:
eine Basis (110);
eine Stütze (130), die vertikal an der Basis (110) angeordnet ist;
einen Traktionsschaft (150), der an der Stütze (130) in senkrechter Richtung vorgesehen ist und dafür ausgelegt ist, in Vorwärts- und Rückwärtsrichtung angetrieben zu werden; **gekennzeichnet durch** ein Doppelgelenkteil (170), das mit dem Traktionsschaft (150) verbunden ist und in Bezug auf eine Antriebsrichtung des Traktionsschafts (150) mehrstufig gebogen werden kann;
wobei das Doppelgelenkteil (170) Folgendes umfasst: ein erstes Kugelgelenk (171), das schwenkbar mit einem Endabschnitt des Traktionsschafts (150) verbunden ist; ein zweites Kugelgelenk (173), das von dem ersten Kugelgelenk (171) beabstandet ist; ein Verbindungselement (175), das das erste und das zweite Kugelgelenk verbindet; und ein Muffenelement (180), das schwenkbar mit dem zweiten Kugelgelenk (173) verbunden und mit einem Patienten verbunden ist.

2. Knochentraktionsvorrichtung (100) nach Anspruch 1, wobei das Verbindungselement (175) aus einer steifen Metallstange oder einem steifen Draht gebildet ist.

3. Knochentraktionsvorrichtung (100) nach Anspruch 1, die ferner Folgendes umfasst: ein Greifelement (190), das in dem Muffenelement (180) installiert ist, um Knochenfragmente des Patienten zu greifen.

4. Knochentraktionsvorrichtung (100) nach Anspruch 3, wobei das Greifelement (190) eine Klemme vom C-Typ ist.

5. Knochentraktionsvorrichtung (100) nach Anspruch 1, die ferner Folgendes umfasst: Luftstiefel (193), die einen Teil eines gebrochenen Abschnitts des Patienten mit Druck umschließen, wobei das Muffenelement (180) mit einem Endabschnitt der Luftstiefel (193) verbunden ist.

6. Knochentraktionsvorrichtung (100) nach Anspruch 1, wobei der Traktionsschaft (150) ein ausfallsicheres Teil umfasst.

7. Knochentraktionsvorrichtung (100) nach Anspruch 1, wobei die Stütze (130) angetrieben werden kann, um vertikal angehoben oder abgesenkt zu werden, um eine Höhe des Traktionsschafts (150) einzustellen.

8. Frakturreduktionsvorrichtung, die Folgendes umfasst:
ein erstes Knochenfragment-Stützteil (200) zum Ergreifen eines proximalen Knochenfragments (B1);
ein zweites Knochenfragment-Stützteil (300) zum Ergreifen eines distalen Knochenfragments (B2);
und
eine Knochentraktionsvorrichtung (100) nach Anspruch 1,
zum Ziehen eines gebrochenen Abschnitts eines Patienten, wobei ein Spitzenabschnitt der Knochentraktionsvorrichtung (100) biegsam geformt ist, um eine Position des distalen Knochenfragments (B2) zu ändern, das von dem zweiten Knochenfragment-Stützteil (300) ergriffen wird.

9. Frakturreduktionsvorrichtung nach Anspruch 8, die ferner Folgendes umfasst: ein Greifelement (190), das in dem Muffenelement (180) installiert ist, um Knochenfragmente des Patienten zu greifen.

10. Frakturreduktionsvorrichtung nach Anspruch 8, die ferner Folgendes umfasst:
Luftstiefel (193), die einen Teil eines gebrochenen Abschnitts des Patienten in einem gepressten Zustand umschließen, wobei das Muffenelement (180) mit einem Endabschnitt der Luftstiefel (193) verbunden ist.

11. Frakturreduktionsvorrichtung nach Anspruch 8, wobei das erste Knochenfragment-Stützteil (200) ein Mehrachsenroboter mit mehreren Freiheitsgraden am proximalen Knochenfragment (B1) ist und das zweite Knochenfragment-Stützteil (300) ein Mehrachsenroboter mit mehreren Freiheitsgraden am distalen Knochenfragment (B2) ist.

12. Frakturreduktionsvorrichtung nach Anspruch 8, wobei das erste Knochenfragment-Stützteil (200) ein mehrachsiger passiver Arm ist, der an einem Operationsbett befestigt ist und am proximalen Knochenfragment (B1) mehrere Freiheitsgrade aufweist,
und das zweite Knochenfragment-Stützteil (200) ein Mehrachsenroboter mit mehreren Freiheitsgraden am distalen Knochenfragment (B2) ist.

13. Frakturreduktionsvorrichtung nach Anspruch 8, wobei das erste und das zweite Knochenfragment-Stützteil mit einem Paar Fixierstiften (201, 203) versehen ist; und mindestens eine Spitze, die das Paar von Befestigungsstiften (201, 203) führt, die an den jeweiligen Knochenfragmenten befestigt werden sollen, und an den jeweiligen Knochenfragmenten an einem Spitzenabschnitt des ersten und zweiten Knochenfragment-Stützteils befestigt ist.

## Revendications

1. Dispositif de traction osseuse (100),
comprenant :
une base (110) ;
un support (130) disposé verticalement sur la base (110) ;
un arbre de traction (150) situé au niveau du support (130) dans une direction perpendiculaire et conçu pour être entraîné dans des directions avant et arrière ; le dispositif étant **caractérisé par** une pièce à double articulation (170) connectée à l'arbre de traction (150) et conçue pour être pliée en de multiples étages par rapport à une direction d'entraînement de l'arbre de traction (150),
la pièce à double articulation (170) incluant : une première articulation à rotule (171) connectée pivotante à une région extrême de l'arbre de traction (150) ; une seconde articulation à rotule (173) espacée de la première articulation à rotule (171) ; un élément de connexion (175) interconnectant les première et seconde articulations à rotule ; et un élément douille (180) connecté pivotant à la seconde articulation à rotule (173) et connecté à un patient.

2. Dispositif de traction osseuse (100) selon la revendication 1, dans lequel l'élément de connexion (175) est formé d'une barre métallique ayant une certaine rigidité ou d'un fil ayant une certaine rigidité.

3. Dispositif de traction osseuse (100) selon la revendication 1, comprenant en outre : un élément de saisie (190) installé dans l'élément douille (180) pour saisir des fragments osseux du patient.

4. Dispositif de traction osseuse (100) selon la revendication 3, dans lequel l'élément de saisie (190) est une pince en C.

5. Dispositif de traction osseuse (100) selon la revendication 1, comprenant en outre : des bottes pneumatiques (193) enfermant avec compression une partie d'une région fracturée du patient, l'élément douille (180) étant connecté à une région extrême des bottes pneumatiques (193).

6. Dispositif de traction osseuse (100) selon la revendication 1, dans lequel l'arbre de traction (150) inclut une partie à sécurité intrinsèque.

7. Dispositif de traction osseuse (100) selon la revendication 1, dans lequel le support (130) est conçu pour être entraîné pour être élevé ou descendu verticalement pour régler une hauteur de l'arbre de traction (150).

8. Appareil de réduction de fracture, comprenant :
une première partie de support de fragment osseux (200) pour saisir un fragment osseux proximal (B1) ;
une seconde partie de support de fragment osseux (300) pour saisir un fragment osseux distal (B2) ; et
un dispositif de traction osseuse (100) selon la revendication 1, pour tracter une région fracturée d'un patient, une région embout du dispositif de traction osseuse (100) étant formée pour être pliable pour changer une position du fragment osseux distal (B2) saisi par la seconde partie de support de fragment osseux (300).

9. Appareil de réduction de fracture selon la revendication 8, comprenant en outre un élément de saisie (190) installé dans l'élément douille (180) pour saisir des fragments osseux du patient.

10. Appareil de réduction de fracture selon la revendication 8, comprenant en outre :
des bottes pneumatiques (193) enfermant dans un état comprimé une partie d'une région fracturée du patient, l'élément douille (180) étant connecté à une région extrême des bottes pneumatiques (193).

11. Appareil de réduction de fracture selon la revendication 8, dans lequel la première partie de support de fragment osseux (200) est un robot à axes multiples ayant de multiples degrés de liberté au niveau du fragment osseux proximal (B1) et dans lequel la seconde partie de support de fragment osseux (300) est un robot à axes multiples ayant de multiples degrés de liberté au niveau du fragment osseux distal (B2).

12. Appareil de réduction de fracture selon la revendication 8, dans lequel la première partie de support de fragment osseux (200) est un bras passif à axes multiples fixé à un lit chirurgical et ayant de multiples degrés de liberté au niveau du fragment osseux proximal (B1) et dans lequel la seconde partie de support de fragment osseux (200) est un robot à axes multiples ayant de multiples degrés de liberté au niveau du fragment osseux distal (B2).

13. Appareil de réduction de fracture selon la revendication 8, dans lequel chacune des première et seconde parties de support de fragment osseux est munie d'une paire de broches de fixation (201, 203) ; et d'au moins une pointe qui guide la paire de broches de fixation (201, 203) à fixer aux fragments osseux respectifs et qui est fixée aux fragments osseux respectifs au niveau d'une région embout des première et seconde parties de support de fragment osseux.
